# EUROPÄISCHE PATENTSCHRIFT

(11) **EP 2 248 487 B1**
(45) Veröffentlichungstag und Bekanntmachung des Hinweises auf die Patenterteilung: **04.11.2015**
(21) Anmeldenummer: 10002311.8
(22) Anmeldetag: 05.03.2010
(51) Int. Cl.: A61F 2/34, A61F 2/30

(54) **Modulare elastische Gelenkpfanne**
Modular elastic joint socket
Poêle articulée modulaire élastique

(30) Priorität: 14.04.2009 DE 102009017406; 01.07.2009 DE 102009031381
(43) Veröffentlichungstag der Anmeldung: 10.11.2010
(73) Patentinhaber: Mathys AG Bettlach, 2544 Bettlach (CH)
(72) Erfinder: Delfosse, Daniel, Dr., 3306 Jegensdorf (CH); Mathys, Hugo, 4571 Lüterkofen (CH); Schenk, Samuel, 3007 Bern (CH)
(74) Vertreter: Körfer, Thomas

(56) Entgegenhaltungen:
- EP-A1- 0 066 092
- EP-A1- 0 297 789
- EP-A1- 0 694 294
- WO-A1-00/64383
- DE-A1- 19 926 923
- FR-A1- 2 733 904
- US-A- 5 458 649

## Beschreibung

Die Erfindung betrifft eine modulare Gelenkpfanne für eine Endoprothese, insbesondere eine Hüftgelenk-Endoprothese zur prothetischen Voll- oder Teilversorgung eines menschlichen oder tierischen Gelenks.

Heutige Hüftgelenk-Endoprothesen haben einen hohen Standard bezüglich ihrer Verweildauer in der geschädigten Hüfte erreicht. Als goldene Regel gilt eine Überlebensrate von 90% nach 10 Jahren, d.h. eine Ausfallsrate von 1% per Jahr. In der zweiten Dekade nach Implantation nimmt die Ausfallrate aber drastisch zu. So gibt es heute keine Gelenkpfannen, die auch nach 20 Jahren noch eine Überlebensrate von 90% oder mehr aufweisen würden.

Gründe für den Ausfall einer Hüftpfanne, insbesondere bei unzementiert implantierten Hüftsystemen sind insbesondere:
- Die Art und Menge der Abriebpartikel, die von den als Artikulationspartnern, das sind Gelenkkugel und Gelenkpfanne, eingesetzten Materialien abgetragen werden, erzeugen Gewebeschädigungen im Körper wie z.B. Osteolysen und führen zu einer aseptischen Lockerung.
- Die Verankerungsoberflächen und die Elastizität der eingesetzten Materialien müssen garantieren, dass der das Implantat umgebende Knochen sich langfristig und dauerhaft mit dem Implantat verbindet, also "osseointegriert", und sich außerdem im Laufe der Zeit nicht durch das so genannte "Stress shielding" zurückbildet.

Im Falle einer notwendigen Re-Operation sollte jedes implantierte System eine einfache Revision gestatten, bevorzugt ausschließlich durch den Ersatz des zerstörten Teiles, normalerweise des weichsten Artikulationspartners.

Als abriebarme und langzeitbeständige Materialien für die Artikulationspartner Gelenkkugel-Gelenkpfanne sind insbesondere Paarungen aus Keramik-Keramik oder Metall-Polyethylen bzw. Keramik-Polyethylen bekannt, wobei das Polyethylen ein Ultra High Molecular Weight Polyethylen (UHMWPE) ist. Das UHMWPE kann hochvernetzt und mit einem Additiv versehen sein.

Die Merkmale des Oberbegriffs des Anspruchs 1 sind aus WO 00/64383 bekannt.

Eine modulare Gelenkpfanne mit Metall-Außenschale und Keramik-Einsatz, wie beispielsweise in der DE 43 37 936 A1 beschrieben, hat den Vorteil, dass im Falle einer Revision, zum Beispiel wegen fortgeschrittenem Abrieb, die gut osseointegrierte Außenschale nicht aus dem Acetabulum herausgerissen werden muss. Stattdessen wird lediglich der Einsatz ausgetauscht, was ohne großen operationstechnischen Aufwand möglich ist. Dafür hat sie den Nachteil, dass auf Grund der hohen Steifigkeit der Metallschale ein großer Teil der Last durch das Implantat aufgenommen und zumeist in einer weit von der Artikulation entfernten Stelle dem umgebenden Knochen wieder übertragen wird. Artikulationsnaher Knochen, z.B. der proximale Femur, wird somit zuwenig belastet und verkümmert ("stress shielding"). Eine dauerhafte Erhaltung der Knochensubstanz kann nicht ausreichend garantiert werden.

Aus der EP 1 728 489 A1 ist eine Monoblock-Gelenkpfanne aus Kunststoff, zum Beispiel aus UHMWPE bekannt, die unzementiert in das Acetabulum eingesetzt wird. Eine solche Polyethylen-Pfanne hat den Vorteil, dass sich auf Grund ihrer hohen Elastizität kein "stress shielding" ausbildet und die Knochensubstanz dauerhaft erhalten bleibt. Dafür hat sie den Nachteil, dass im Falle einer Revision, zum Beispiel wegen fortgeschrittenem Abrieb, die gesamte, gut osseointegrierte Pfanne aus dem Acetabulum herausgerissen werden muss.

Es ist daher die Aufgabe der Erfindung, eine Gelenkpfanne für eine Endoprothese zu beschreiben, die eine Rückbildung des umgebenden Knochens durch "stress shielding" minimiert und modular aufgebaut ist um einen einfachen Austausch nur des durch Abrieb zerstörten Teils des Implantats zu ermöglichen. Eine gut osseointegrierte Außenschale soll dabei im Knochen verbleiben können.

Die Aufgabe wird durch die erfindungsgemäße Gelenkpfanne gemäß Anspruch 1 gelöst. In den Unteransprüchen sind vorteilhafte Weiterbildungen der erfindungsgemäßen Gelenkpfanne dargestellt.

Die erfindungsgemäße modulare Gelenkpfanne für eine Endoprothese, die mit einer Gelenkkugel eine Gleitpaarung bildet, umfasst eine Außenschale sowie einen mit der Außenschale verbundenen Einsatz, wobei die Außenschale aus einem elastischen Material aufgebaut ist und der Einsatz lösbar mit der Außenschale verbunden ist.

Eine Außenschale aus elastischem Material ermöglicht eine physiologische Lastübertragung auf den umgebenden Knochen und verhindert somit die Rückbildung des artikulationsnahen Knochens. Der lösbar mit der Außenschale verbundene Einsatz ermöglicht ein Entfernen des Einsatzes, ohne die gut osseointegrierte Außenschale aus dem Knochen reißen zu müssen. Damit wird eine weitere Abtragung des Acetabulum verhindert, der operative Eingriff ist weniger belastend für den Patienten und die Kosten für das ausgetauschte Implantat und die Operation selbst werden reduziert.

Erfindungsgemäß ist die Außenschale aus einem elastischen Material mit einem Elastizitätsmodul entsprechend dem Elastizitätsmodul eines spongiösen Knochenmaterials aufgebaut. Dies ermöglicht eine optimale Anpassung und Kraftübertragung der Außenschale an den umgebenden Knochen und unterstützt somit den dauerhaften Erhalt des Acetabulums und einen festen Sitz der Außenschale im Knochen. Dabei sollte der Elastizitätsmodul des Materials der Außenschale kleiner 3GPa sein. Der Einsatz ist vorteilafterweise ebenso aus einem elastischen Material wie die Außenschale oder aus Keramik ausgebildet und bildet somit eine abriebarme, langlebige Gleitpaarung mit üblicherweise eingesetzten Gelenkköpfen aus Metal oder Keramik.

Vorteilhafterweise ist zwischen Außenschale und Einsatz ein Verbundelement eingesetzt. Alternativ bildet der Einsatz ein monolithisches Schnappelement aus und stellt darüber eine Verbindung mit der Außenschale her. Da sich glatte elastische Körper nur schwer miteinander verklemmen lassen, ermöglicht ein Schnappelement oder ein Verbundelement wie z.B. eine Metallhülse, ein Spannring oder Segering gute gegenseitige Befestigung aneinander. Ein Verbundelement aus metallischem Material wird außerdem in Röntgenaufnahmen gut abgebildet und ermöglicht somit die Überprüfung der Lage und der Ausrichtung des Implantats im Acetabulum.

Die Metallhülse weist vorteilhafter Weise Drehrillen und/oder Schlitze auf, sodass auf einfache Weise eine feste und verdrehsichere Verbindung zwischen Einsatz und Außenschale gegeben ist. Außerdem wird die Metallhülse durch Schlitze_oder auch andere geometrische Maßnahmen elastischer und verringert damit die Elastizität des Gesamtsystems weniger stark als eine ungeschlitzte Metallhülse.

Zur optimalen physiologischen Kraftübertragung an die Außenschale ist der Einsatz als Halbkugel oder Stumpfkegel oder in Stufenform ausgebildet. Ist der Einsatz als Stumpfkegel ausgeformt, weist der Kegelwinkel vorteilhafter Weise einen Wert zwischen 1° und 10°, vorzugsweise zwischen 3° und 7°, besonders bevorzugt von 5° auf. Ist der Einsatz mittels einer Metallhülse in der Außenschale verankert und der Einsatz als Stumpfkegel ausgeformt, so liegt der Kegelwinkel des Stumpfkegels vorteilhafter Weise zwischen 5° und 30°, bevorzugt zwischen 15° und 20°, besonders bevorzugt zwischen 18° und 19°. Ein Einsatz aus Keramik ist vorteilhafter Weise als Stumpfkegel mit einem Kegelwinkel zwischen 5° und 20°, bevorzugt zwischen 15° und 20°, besonders bevorzugt zwischen 18° und 19°, ausgebildet.

In einer anderen Ausführungsform ist der Einsatz mittels Schnapp- oder Bajonett- oder Schraubverbindung in der Außenschale verankert. Dies ermöglicht in vorteilhafter Weise eine einfache, schnelle verdreh- und rutschsichere Befestigung des Einsatzes an der Außenschale. Durch Verwendung eines einfachen Werkzeugs lassen sich solche Verbindungen lösen ohne die Außenschale zu beschädigen. Insbesondere kann eine Beschädigung der Außenschale vermieden werden, wenn eine Schnappverbindung in der Außenschale starr und im Einsatz elastisch gestaltet ist. Die Außenschale der erfindungsgemäßen Gelenkpfanne ist vorteilhafter Weises aus einem Polymer, bevorzugt aus Polyethylen oder aus einem Verbundwerkstoff aufgebaut. Diese Materialien erlauben eine gute Anpassung ihrer elastischen Eigenschaften an den Elastizitätsmodul des spongiösen Knochens, sodass eine gleichmäßige Kraftübertragung gewährleistet ist.

Von Vorteil ist ebenfalls, dass die dem Knochen zugewandte Oberfläche der Außenschale mit Partikeln oder Netzen oder Schäumen aus Titan oder Tantal oder Kalziumphosphat beschichtet ist. Titan oder Tantal oder Kalziumphosphat sind inerte, bioaktive Materialien und bilden als Partikel, netz- oder schaumartig auf der Oberfläche der Außenschale aufgebracht, eine stark aufgeraute oder poröse Oberfläche, die sich besonders gut und dauerhaft mit dem umgebenden Knochen verbindet.

Vorteilhafter Weise ist der erfindungsgemäße Verbundwerkstoff durch eine Armierung, Fasern oder Partikel, auch Nanopartikel, verstärkt, insbesondere mit Aramid- oder Polyethylen- oder Karbon- oder Bioglas-Fasern. Damit bleibt das Material auch unter den starken Druck- und Stoßbelastung, denen die Gelenkpfanne ausgesetzt ist, dauerhaft formstabil und bruchsicher.

Vorteilhafter Weise besteht der Einsatz aus dem gleichen Material wie die Außenschale. Das erfindungsgemäße Material der Außenschale weist eine hervorragende Gleitpaarung mit einem Gelenkkopf aus Metall oder Keramik auf. Anderseits ist die Herstellung der Außenschale und des Einsatzes aus dem gleichen Material kostengünstig, da das gleiche Material mit gleichartigen Maschinen und gleicher Bearbeitungstechnik bearbeitet werden kann. Vorteilhafter Weise besteht der Einsatz aus elastischem UHMWPE, insbesondere hochvernetztes UHMWPE. Das stabilisierte und hochvernetzte UHMWPE ist besonders alterungs- und oxidationsbeständig und versprödet nicht, sodass gewährleistet ist, dass über einen Zeitraum nur sehr wenig Abreibpartikel generiert werden.

Ausführungsbeispiele der erfindungsgemäßen Gelenkpfanne sind in den Zeichnungen beispielhaft dargestellt und werden anhand der nachfolgenden Beschreibung näher erläutert. Es zeigen:
- Fig.1: eine erste erfindungsgemäße modulare Gelenkpfanne mit Metallhülse in perspektivischer Ansicht;
- Fig.2: die erste erfindungsgemäße modulare Gelenkpfanne mit Metallhülse im Schnitt;
- Fig.3: ein erstes Ausführungsbeispiel einer erfindungsgemäßen Metallhülse in perspektivischer Ansicht;
- Fig.4: ein zweites Ausführungsbeispiel einer erfindungsgemäßen Metallhülse in perspektivischer Ansicht;
- Fig.5: einen Randbereich der ersten erfindungsgemäßen modularen Gelenkpfanne im Schnitt;
- Fig.6: eine zweite erfindungsgemäße modulare Gelenkpfanne mit Spannring in Blickrichtung entlang der Symmetrieachse;
- Fig.7: die zweite erfindungsgemäße modulare Gelenkpfanne mit Spannring im Schnitt;
- Fig.8: einen erfindungsgemäßen Einsatz einer zweiten modularen Gelenkpfanne in perspektivischer Ansicht;
- Fig.9: einen erfindungsgemäßen Spannring einer zweiten modularen Gelenkpfanne in perspektivischer Ansicht;
- Fig.10: einen Randbereich der zweiten erfindungsgemäße modulare Gelenkpfanne im Schnitt;
- Fig.11: eine dritte erfindungsgemäße modulare Gelenkpfanne mit Segering in Blickrichtung entlang der Symmetrieachse;
- Fig.12: die dritte erfindungsgemäße modulare Gelenkpfanne mit Segering im Schnitt;
- Fig.13: eine erfindungsgemäße Außenschale der dritten modularen Gelenkpfanne in perspektivischer Ansicht;
- Fig.14: einen erfindungsgemäßen Einsatz des dritten Ausführungsbeispiel einer modularen Gelenkpfanne in perspektivischer Ansicht;
- Fig.15: einen Randbereich der dritten erfindungsgemäßen modulare Gelenkpfanne im Schnitt;
- Fig.16: eine erfindungsgemäße Außenschale einer vierten modularen Gelenkpfanne in perspektivischer Ansicht;
- Fig.17: eine vierte erfindungsgemäße modulare Gelenkpfanne mit Schnappelement im Schnitt;
- Fig.18: einen erfindungsgemäßen Einsatz der vierten modularen Gelenkpfanne mit Schnappelement in perspektivischer Ansicht;
- Fig.19: einen erfindungsgemäßen Einsatz der vierten modularen Gelenkpfanne mit geschlitztem Schnappelement in perspektivischer Ansicht und
- Fig.20: einen vergrößerten Ausschnitt des geschlitzten Schnappelements der vierten erfindungsgemäßen modulare Gelenkpfanne in perspektivischer Ansicht.

Einander entsprechende Teile sind in allen Figuren mit den gleichen Bezugszeichen versehen.

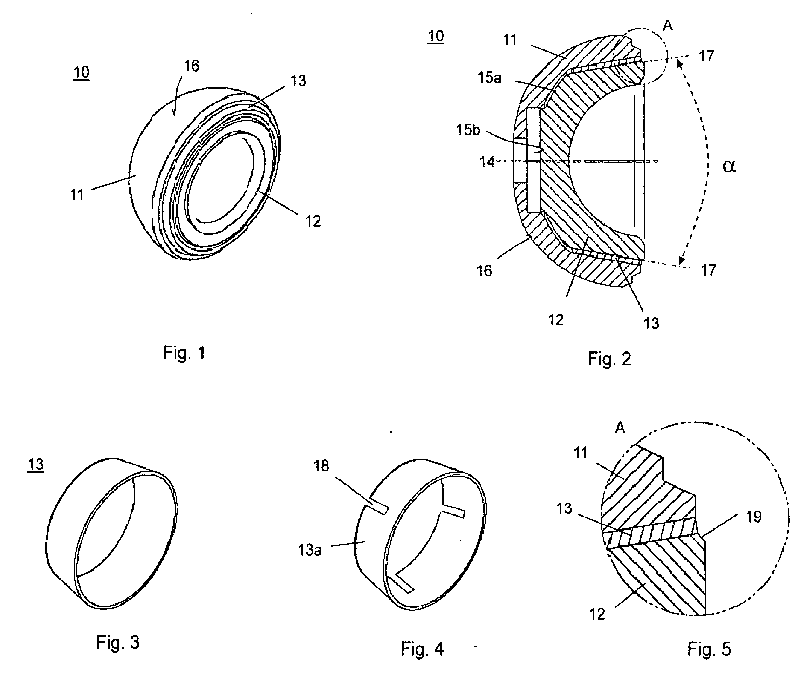

Fig.1 bis Fig.5 zeigen ein erstes Ausführungsbeispiel der erfindungsgemäßen modularen Gelenkpfanne, die aus einer Außenschale 11 mit integrierter Metallhülse 13 und einem einfach entfernbaren Einsatz 12 zusammengesetzt ist. Die Außenschale 11 besteht bevorzugt aus Polyethylen oder einem Kunststoff-Komposit, das auf geringe strukturelle Steifigkeit optimiert ist. Die dem Acetabulum zugewandten Außenseite 16 der Außenschale 11 ist bevorzugt mit Partikeln oder Netzen oder Schäumen aus Titan oder Tantal oder Kalziumphosphat beschichtet. Dies gewährleistet einerseits die dauerhafte Osseointegration, hat andererseits aber keinen wesentlichen Einfluss auf die hohe Elastizität des Verbundes aus Außenschale 11 und Einsatz 12. Ein Einsatz 12 besteht aus Polyethylen, insbesondere dem z. B. durch γ-Strahlung oder durch Röntgen-Strahlung (X-linked) hochvernetzten UHMWPE, welches durch den Zusatz eines Antioxidanten (z. B. Vitamin E) äußerst alterungsbeständig wird.

Der Einsatz 12 ist als Stumpfkegel mit einem gewölbten Dach 15a, das zur Symmetrieachse 14 der Gelenkpfanne hin in gerades Dach 15b übergeht, ausgebildet, wie in Fig.2 deutlich zu sehen ist. Der Kegelwinkel α, der von der Mantelfläche 17 aufgespannt wird, liegt in einem Bereich zwischen 5° und 30°, bevorzugt zwischen 15° und 20°, besonders bevorzugt zwischen 18° und 19°. Dadurch ist eine gute Kraftübertragung zwischen Einsatz 12, Metallhülse 13 und Außenschale 11 gegeben. Alternativ kann der Einsatz 12 als Halbkugel mit geradem oder gewölbtem Dach geformt sein. Die Außenschale 11 ist an ihrer Innenseite gegengleich ausgeformt und nimmt den Einsatz passgenau auf.

Da sich Kunststoffe, insbesondere Polyethylen auf Polyethylen, nur unzureichend gegeneinander verklemmen, ist zur verrutschsicheren Verankerung eine Metallhülse 13 bzw. 13a zwischen Außenschale 11 und Einsatz 12 eingesetzt. Wie in den Ausführungsbeispielen in Fig. 3 und 4 dargestellt, verjüngt sich diese ebenfalls mit dem Kegelwinkel α. Die Metallhülse 13a aus Fig.4 ist durch eine Mehrzahl von axial verlaufenden Schlitzen 18 oder Drehrillen oder andere geometrische Ausnehmungen elastischer als eine gleich dimensionierte Metallhülse 13 ohne Schlitze und verringert weniger die Elastizität des Gesamtsystems. Die Schlitze 18 können auch von beiden Seiten in die Hülse eingeschnitten sein.

Fig.5 zeigt vergrößert den Randbereich A aus Fig. 2 der ersten Gelenkpfanne 10. Die Außenschale 11 schließt bündig mit der Metallhülse 13 ab. Der Einsatz 12 steht dagegen um einen Überstand 19 über die Hülse heraus. Zur Revision des Einsatzes 12 wird mit einem Werkzeug an diesem Überstand 19 angesetzt, der Einsatz 12 zusammengedrückt und somit von der Metallhülse 13, 13a und der Außenschale 11 gelöst. Zur lösbaren Verankerung kann aber auch ein Bajonett-, Schnapp- oder Gewindeverschluss im Randbereich ausbildet sein.

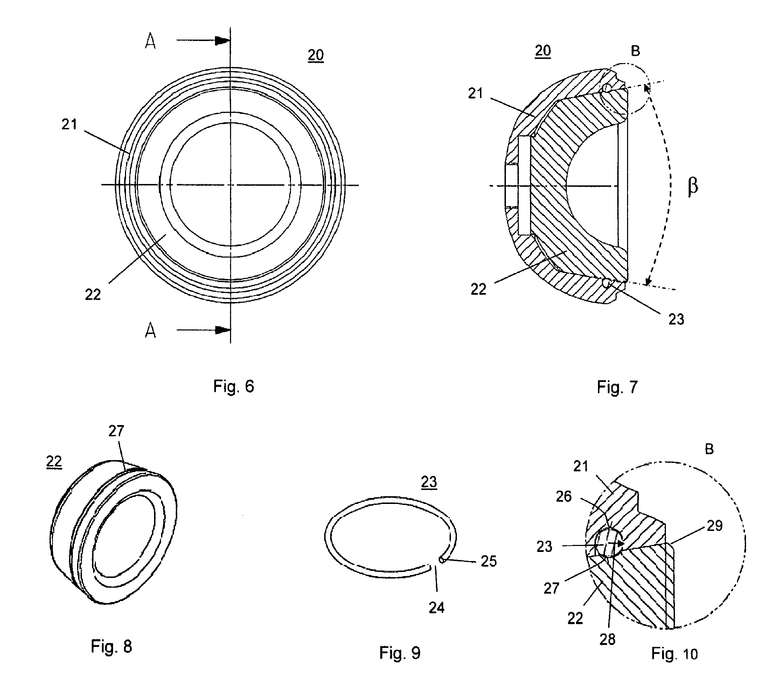

Fig. 6 bis 10 zeigen ein zweites Ausführungsbeispiel 20 einer modularen Gelenkpfanne, bei dem die Außenschale 21 und der Einsatz 22 durch einen Spannring 23 miteinander lösbar verbunden sind. Material und Oberflächenstruktur der Außenschale sowie das Material des Einsatzes 21 entsprechen denen des ersten Ausführungsbeispiels. Fig. 6 zeigt die modulare Gelenkpfanne 20 in Blickrichtung entlang der Symmetrieachse 14, Fig. 7 einen Schnitt entlang der Achse A-A. Da in der modularen Gelenkpfanne 20 ein Spannring verwendet wird, ist bei der Wahl des Kegelwinkels β des Einsatzes 22 und der Innenseite der Außenschale keine Eingrenzung auf einen bestimmten Wert nötig, um eine gute Verbindung und Kraftübertragung zu gewährleisten.

Ein Spannring 23 mit rundem Profil 25 und einer Öffnung 24, wie in Fig. 9 zu sehen, ist im Randbereich B zwischen Außenschale 21 und Einsatz 22 eingelegt. Wie im vergrößert dargestellten Randbereich B der Fig. 7 der zweiten Gelenkpfanne 20 in Fig. 10 dargestellt, weisen die Innenseite der Außenschale 21 und die Außenseite des Einsatzes 22 umfänglich jeweils eine Nute 26 bzw. 27 mit rundem Profil auf, deren Radien dem Radius 28 des leicht zusammengedrückten Spannringprofils 25 entsprechen. Zum Einlegen wird der Spannring 23 zusammengedrückt und in die Nut 26 der Außenschale 21 eingespannt. Die Nut 27 im elastischen Einsatz 22, siehe auch Fig. 8, greift beim Einsetzen über den Spannring 23 in der Außenschale 21 und schafft somit eine stabile Verankerung.

Zum Herauslösen des Einsatzes 22 aus der Außenschale 21 greift ein Werkzeug am Überstand 29 an, drückt den Einsatz 22 zusammen und löst somit den Einsatz 22 vom Spannring 23. Die Außenschale 21 verbleibt unversehrt und fest im Acetabulum verankert.

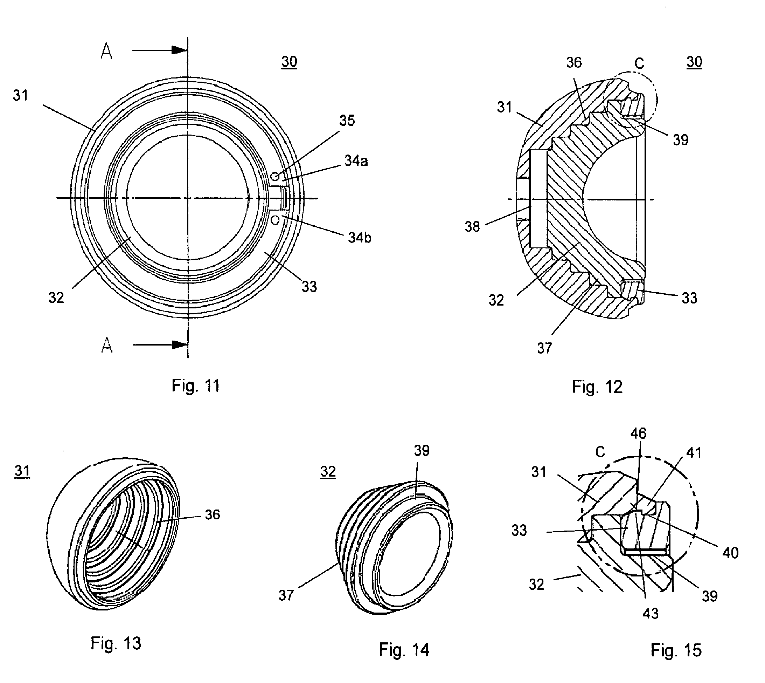

Die Fig. 11 bis 15 zeigen ein drittes Ausführungsbeispiel einer erfindungsgemäßen modularen Gelenkpfanne. Wie in Fig.11 dargestellt, ist der Einsatz 32 über einen Segering 33 mit der Außenschale 31 verbunden. Die Innenseite 36 der Außenschale 31 und die Außenseite 37 des Einsatzes 32 weisen eine Stufenform mit einem geraden Dach 38 auf. Dies ist in der perspektivischen Ansicht der Außenschale 31 in Fig. 13 und des Einsatzes in Fig. 14 gut zu erkennen. Auch durch die Stufenform ist eine gute Kraftübertragung vom Einsatz 32 zur Außenschale 31 gegeben.

Wie Fig. 12 und vergrößert Fig. 15 zeigen, weist der Randbereich C des Einsatzes 32 eine umfängliche Ausnehmung 39 auf. Am äußeren Rand der Außenschale 31 ist eine Nase 41 ausgebildet. Ist der Einsatz 32 in die Außenschale 31 eingesetzt, wird der Segering 33 zwischen Außenschale 31 und Einsatz 32 in die Ausnehmung 39 eingeschoben, sodass die Nase 41 in die umfängliche Nut 40 des Segerings 33 und der Überstand 43 des Segerings in die umfängliche Nut 46 der Außenschale 31 einrastet.

Zum Entfernen des Einsatzes 32 greift ein Werkzeug in die Durchgangsbohrungen 35 im Segering 33 ein, verkleinert durch Zusammenführen der Enden 34a, 34b des Segerings 33 dessen Radius und löst die Nut 40 von der Nase 41. Der Segering 33 wird abgezogen und der Einsatz 32 kann von der Außenschale 31 gelöst werden.

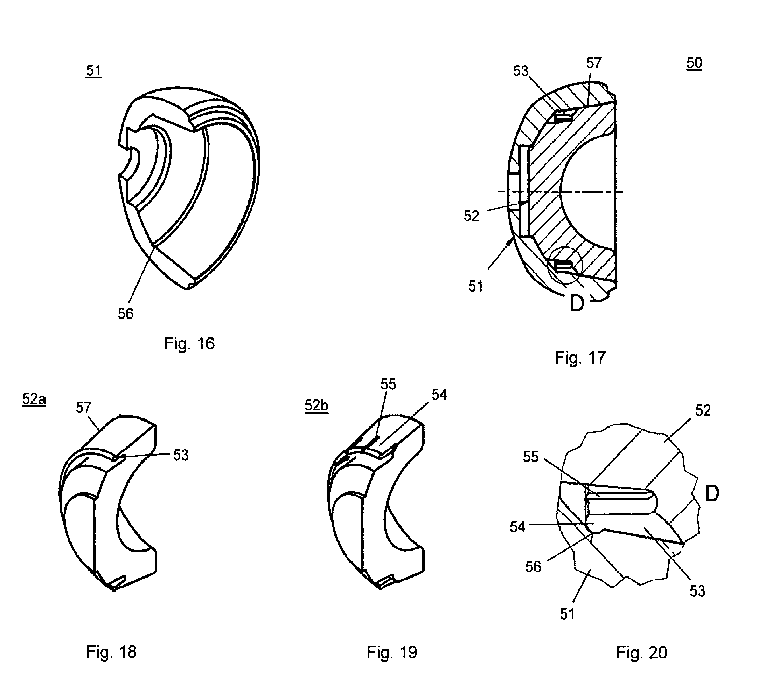

Die Fig. 16 bis 20 zeigen ein viertes Ausführungsbeispiel einer erfindungsgemäßen modularen Gelenkpfanne. Dabei sind der Einsatz 52 und die Außenschale 51 durch eine Schnappverbindung D, wie in Fig. 17 und 20 dargestellt, verbunden. An der Außenseite 57 des Einsatzes 52 ist auf etwa halber axialer Höhe ein Schnappelement 53 aus dem gleichen Material, z.B. UHMWPE, wie der Einsatz selbst ausgeformt. Das Schnappelement 53 erstreckt sich über den gesamten Umfang des Einsatzes 52 und ist entweder als ein ununterbrochener Ring, siehe Einsatz 52a in Fig. 15 oder als ein durch Schlitze 55 unterbrochener Ring, siehe Einsatz 52b, ausgebildet. In Fig. 20 ist die Schnappverbindung D am Beispiel des Einsatzes 52b vergrößert dargestellt. Eine zungenförmige Fortsetzung des Einsatzes 52 bildet das Schnappelement 53, das in einer kugelförmig verdickten Zungenspitze 54 ausläuft. Diese Zungenspitze 54 rastet in eine umfänglich an der Innenseite der Außenschale 51 verlaufende Nut 56 ein. Axial verlaufende Schlitze 55 im Schnappelement 53 erleichtern ein Zusammendrücken des Schnappelements 53 in axiale Richtung beim Einsetzen in die Außenschale 51.

Ein erfindungsgemäßer Einsatz kann auch mittels einer Bajonett- oder Schraubverbindung mit der Außenschale verankert werden. In allen Verankerungsvarianten bleibt die Außenschale 11, 21, 31, 51 starr. Die Verbindung wird durch die elastische Eigenschaft des Einsatzes 12, 22, 32, 52 gelöst.

Der Einsatz einer erfindungsgemäßen modularen Gelenkpfanne kann auch aus Keramik bestehen und als Halbkugel oder als Stumpfkegel mit geradem oder gewölbtem Dach geformt sein. Bei einem Stumpfkegel liegt der Kegelwinkel α zwischen 5° und 20°, bevorzugt zwischen 18° und 19°. Der Keramikeinsatz ist lösbar mit der Außenschale über einen Bajonett-, Schnapp- oder Gewindeverschluss verbunden. Von Vorteil ist auch, dass eine für die jeweilige Situation günstige Außenschale mit einem entsprechenden Einsatz interoperativ zugepaart werden können.

Alle beschriebenen und/oder gezeichneten Merkmale können im Rahmen der Erfindung vorteilhaft miteinander kombiniert werden. Die Erfindung ist nicht auf die Ausführungsbeispiele beschränkt und wird durch die Ansprüche definiert. Als Kunststoffe kommen alle thermoplastischen Kunststoffe, PEEK und Epoxide in Betracht.

## Patentansprüche

1. Modulare Gelenkpfanne (10) für eine Endoprothese, die mit einer Gelenkkugel eine Gleitpaarung bildet, mit einer Außenschale (11) sowie einem mit der Außenschale (11) verbundenen Einsatz (12), wobei die Außenschale (11) aus einem elastischen Material besteht und der Einsatz (12) lösbar mit der Außenschale (11) verbunden ist,
**dadurch gekennzeichnet,**
**dass** die Außenschale (11) aus einem elastischen Material mit einem Elastizitätsmodul entsprechend dem Elastizitätsmodul eines spongiösen Knochenmaterials besteht.

2. Modulare Gelenkpfanne nach Anspruch 1,
**dadurch gekennzeichnet,**
**dass** der Einsatz (12) aus einem ebenso elastischen Material oder aus Keramik ausgebildet ist.

3. Modulare Gelenkpfanne nach Anspruch 1 oder 2,
**dadurch gekennzeichnet,**
**dass** zwischen Außenschale (11) und Einsatz (12) ein Verbundelement eingesetzt ist.

4. Modulare Gelenkpfanne nach Anspruch 1 oder 2,
**dadurch gekennzeichnet,**
**dass** der Einsatz (12) ein monolithisches Schnappelement (53) ausbildet und sich über das Schnappelement (53) mit der Außenschale (11) verbindet.

5. Modulare Gelenkpfanne nach Anspruch 3,
**dadurch gekennzeichnet,**
**dass** das Verbundelement als Metallhülse (13) und/oder als Spannring (23) und/oder als Segering (33)ausgeformt ist.

6. Modulare Gelenkpfanne nach Anspruch 3 oder 5,
**dadurch gekennzeichnet,**
**dass** das Verbundelement aus einem metallischen Material, bevorzugt aus Titan, Kobalt-Chrom oder rostfreiem Stahl besteht.

7. Modulare Gelenkpfanne nach Anspruch 5 oder 6,
**dadurch gekennzeichnet,**
**dass** die Metallhülse (13) Drehrillen und/oder Schlitze (18) aufweist.

8. Modulare Gelenkpfanne nach einem der Ansprüche 1 bis 7,
**dadurch gekennzeichnet,**
**dass** der Einsatz (12) als Halbkugel oder Stumpfkegel oder in Stufenform ausgebildet ist.

9. Modulare Gelenkpfanne nach Anspruch 8,
**dadurch gekennzeichnet,**
**dass** der Kegelwinkel (β) eines als Stumpfkegel ausgeformten Einsatzes (22) zwischen 1° und 10°, vorzugsweise zwischen 3° und 7°, besonders bevorzugt bei 5° liegt.

10. Modulare Gelenkpfanne nach Anspruch 8,
**dadurch gekennzeichnet,**
**dass** der Einsatz (12) mittels einer Metallhülse (13) in der Außenschale (11) verankert ist und der Einsatz (12) als Stumpfkegel mit einem Kegelwinkel (α) zwischen 5° und 30°, bevorzugt zwischen 15° und 20°, besonders bevorzugt zwischen 18° und 19°, ausgebildet ist.

11. Modulare Gelenkpfanne nach Anspruch 8,
**dadurch gekennzeichnet,**
**dass** ein Einsatz (12) aus Keramik als Stumpfkegel mit einem Kegelwinkel zwischen 5° und 20°, bevorzugt zwischen 15° und 20°, besonders bevorzugt zwischen 18° und 19°, ausgebildet ist.

12. Modulare Gelenkpfanne nach einem der Ansprüche 1 bis 11,
**dadurch gekennzeichnet,**
**dass** der Einsatz (12) mittels Schnapp- oder Bajonett- oder Schraubverbindung in der Außenschale (11) verankert ist.

13. Modulare Gelenkpfanne nach Anspruch 12,
**dadurch gekennzeichnet,**
**dass** die Schnappverbindung in eine Nut (26, 46, 56) in der Außenschale (11) eingreift.

14. Modulare Gelenkpfanne nach Anspruch 12 oder 13,
**dadurch gekennzeichnet,**
**dass** eine Schnappverbindung in der Außenschale (11) starr und im Einsatz (12) elastisch gestaltet ist.

15. Modulare Gelenkpfanne nach einem der Ansprüche 1 bis 14,
**dadurch gekennzeichnet,**
**dass** die Außenschale (11) aus einem Polymer, bevorzugt aus Polyethylen oder UHMW-Polyethylen oder einem Verbundwerkstoff oder einem mit Nanopartikeln verstärkten Kunststoff besteht.

16. Modulare Gelenkpfanne nach einem der Ansprüche 1 bis 15,
**dadurch gekennzeichnet,**
**dass** die dem Knochen zugewandten Oberfläche (16) der Außenschale (11) mit Partikeln oder Netzen oder Schäumen aus Titan oder Tantal oder Kalziumphosphat beschichtet ist.

17. Modulare Gelenkpfanne nach Anspruch 15,
**dadurch gekennzeichnet,**
**dass** der Verbundwerkstoff durch eine Armierung, Fasern oder Partikel verstärkt ist, insbesondere mit Aramid- oder Polyethylen- oder Karbon- oder Bioglas-Fasern.

18. Modulare Gelenkpfanne nach einem der Ansprüche 15 oder 17,
**dadurch gekennzeichnet,**
**dass** der Einsatz (12) aus dem gleichen Material wie die Außenschale (11) besteht.

19. Modulare Gelenkpfanne nach einem der Ansprüche 15 oder 17 oder 18,
**dadurch gekennzeichnet,**
**dass** der Einsatz (12) aus elastischem UHMW-Polyethylen besteht.

## Claims

1. Modular joint socket (10) for an endoprosthesis which forms a sliding pairing with a joint ball, with an outer shell (11) and an insert (12) connected with the outer shell (11), wherein the outer shell (11) consists of an elastic material and the insert (12) is connected detachably with the outer shell (11),
**characterised in that**
the outer shell (11) consists of an elastic material with a modulus of elasticity corresponding to the modulus of elasticity of a spongiose bone material.

2. Modular joint socket according to claim 1,
**characterised in that**
the insert (12) is made of an equally elastic material or of ceramic.

3. Modular joint socket according to claim 1 or 2,
**characterised in that**
a composite element is inserted between the outer shell (11) and the insert (12).

4. Modular joint socket according to claim 1 or 2,
**characterised in that**
the insert (12) comprises a monolithic snap element (53) and is connected by means of the snap element (53) with the outer shell (11).

5. Modular joint socket according to claim 3,
**characterised in that**
the composite element is embodied as a metal sleeve (13) and/or as a clamping ring (23) and/or as a circlip ring (33).

6. Modular joint socket according to claim 3 or 5,
**characterised in that**
the composite element consists of a metallic material, preferably of titanium, cobalt-chromium or stainless steel.

7. Modular joint socket according to claim 5 or 6,
**characterised in that**
the metal sleeve (13) exhibits grooves and/or slots (18).

8. Modular joint socket according to one of claims 1 to 7,
**characterised in that**
the insert (12) is embodied as a hemisphere or truncated cone or a step shape.

9. Modular joint socket according to claim 8,
**characterised in that**
the cone angle (β) of an insert (22) embodied as a truncated cone is between 1° and 10°, preferably between 3° and 7°, particularly preferably 5°.

10. Modular joint socket according to claim 8,
**characterised in that**
the insert (12) is anchored in the outer shell (11) by means of a metal sleeve (13) and the insert (12) is embodied as a truncated cone with a cone angle (α) between 5° and 30°, preferably between 15° and 20°, particularly preferably between 18° and 19°.

11. Modular joint socket according to claim 8,
**characterised in that**
an insert (12) of ceramic is embodied as a truncated cone with a cone angle between 5° and 20°, preferably between 15° and 20°, particularly preferably between 18° and 19°.

12. Modular joint socket according to one of claims 1 to 11,
**characterised in that**
the insert (12) is anchored in the outer shell (11) by means of a snap or bayonet or screw connection.

13. Modular joint socket according to claim 12,
**characterised in that**
the snap connection engages in a groove (26, 46, 56) in the outer shell (11).

14. Modular joint socket according to claim 12 or 13,
**characterised in that**
a snap connection is formed rigidly in the outer shell (11) and elastically in the insert (12).

15. Modular joint socket according to one of claims 1 to 14,
**characterised in that**
the outer shell (11) consists of a polymer, preferably of polyethylene or UHMW (ultra-high molecular weight) polyethylene or a composite material or a plastic reinforced with nano-particles.

16. Modular joint socket according to one of claims 1 to 15,
**characterised in that**
the surface (16) of the outer shell (11) facing the bone is coated with particles or networks or foams of titanium or tantalum or calcium phosphate.

17. Modular joint socket according to claim 15,
**characterised in that**
the composite material is strengthened by a reinforcement, fibres or particles, in particular with aramide or polyethylene or carbon or bioglass fibres.

18. Modular joint socket according to one of claims 15 or 17,
**characterised in that**
the insert (12) consists of the same material as the outer shell (11).

19. Modular joint socket according to one of claims 15 or 17 or 18,
**characterised in that**
the insert (12) consists of elastic UHMW polyethylene.

## Revendications

1. Cupule d'articulation (10) modulaire pour une endoprothèse, qui forme avec une rotule d'articulation, un couple d'articulation à glissement, comprenant une coque extérieure (11) ainsi qu'un insert (12) relié à la coque extérieure (11), la coque extérieure (11) étant réalisée en un matériau élastique, et l'insert (12) étant relié de manière amovible à la coque extérieure (11),
**caractérisée**
**en ce que** la coque extérieure (11) est réalisée en un matériau élastique présentant un module d'élasticité correspondant au module d'élasticité d'une matière osseuse spongieuse.

2. Cupule d'articulation modulaire selon la revendication 1,
**caractérisée**
**en ce que** l'insert (12) est réalisé en un matériau également élastique ou en céramique.

3. Cupule d'articulation modulaire selon la revendication 1 ou la revendication 2,
**caractérisée**
**en ce qu'**entre la coque extérieure (11) et l'insert (12) est inséré un élément de liaison d'assemblage.

4. Cupule d'articulation modulaire selon la revendication 1 ou la revendication 2,
**caractérisée**
**en ce que** l'insert (12) forme un élément à enclenchement (53) monolithique, et s'assemble avec la coque extérieure (11) par l'intermédiaire de l'élément à enclenchement (53).

5. Cupule d'articulation modulaire selon la revendication 3,
**caractérisée**
**en ce que** l'élément de liaison d'assemblage est configuré sous la forme d'une douille métallique (13) et/ou d'un anneau de serrage du type jonc d'arrêt (23) et/ou d'un anneau élastique du type circlips (33).

6. Cupule d'articulation modulaire selon la revendication 3 ou la revendication 5,
**caractérisée**
**en ce que** l'élément de liaison d'assemblage est réalisé en un matériau métallique, de préférence en titane, chrome-cobalt ou en acier inoxydable.

7. Cupule d'articulation modulaire selon la revendication 5 ou la revendication 6,
**caractérisée**
**en ce que** la douille métallique (13) présente des stries de rotation et/ou des fentes (18).

8. Cupule d'articulation modulaire selon l'une des revendications 1 à 7,
**caractérisée**
**en ce que** l'insert (12) est réalisé en tant que demi-sphère, ou de tronc de cône ou selon une forme étagée.

9. Cupule d'articulation modulaire selon la revendication 8,
**caractérisée**
**en ce que** l'angle de cône (β) d'un insert (22) réalisé sous forme de tronc de cône, se situe entre 1° et 10°, de préférence entre 3° et 7°, de manière particulièrement préférée aux alentours de 5°.

10. Cupule d'articulation modulaire selon la revendication 8,
**caractérisée**
**en ce que** l'insert (12) est ancré au moyen d'une douille métallique (13) dans la coque extérieure (11), et l'insert (12) est réalisé sous forme de tronc de cône avec un angle de cône (α) entre 5° et 30°, de préférence entre 15° et 20°, et de manière particulièrement préférée entre 18° et 19°.

11. Cupule d'articulation modulaire selon la revendication 8,
**caractérisée**
**en ce qu'**un insert (12) en céramique est réalisé en tant que tronc de cône avec un angle de cône entre 5° et 20°, de préférence entre 15° et 20°, et de manière particulièrement préférée entre 18° et 19°.

12. Cupule d'articulation modulaire selon l'une des revendications 1 à 11,
**caractérisée**
**en ce que** l'insert (12) est ancré dans la coque extérieure (11) au moyen d'une liaison à enclenchement, à baïonnette ou à vis.

13. Cupule d'articulation modulaire selon la revendication 12,
**caractérisée**
**en ce que** la liaison par enclenchement s'engage dans une rainure (26, 46, 56) dans la coque extérieure (11).

14. Cupule d'articulation modulaire selon la revendication 12 ou la revendication 13,
**caractérisée**
**en ce qu'**une liaison par enclenchement est d'une configuration rigide dans la coque extérieure (11) et élastique dans l'insert (12).

15. Cupule d'articulation modulaire selon l'une des revendications 1 à 14,
**caractérisée**
**en ce que** la coque extérieure (11) est réalisée en un polymère, de préférence en polyéthylène ou polyéthylène UHMW, ou en un matériau composite, ou une matière plastique renforcée de nanoparticules.

16. Cupule d'articulation modulaire selon l'une des revendications 1 à 15,
**caractérisée**
**en ce que** la surface (16) de la coque extérieure (11), qui est dirigée vers l'os, est revêtue de particules ou de réseaux ou de mousses en titane ou tantale ou en phosphate de calcium.

17. Cupule d'articulation modulaire selon la revendication 15,
**caractérisée**
**en ce que** le matériau composite est renforcé par une armature, des fibres ou des particules, notamment par des fibres d'aramide ou de polyéthylène ou de carbone ou bio-verre.

18. Cupule d'articulation modulaire selon l'une des revendications 15 ou 17,
**caractérisée**
**en ce que** l'insert (12) est réalisé dans le même matériau que la coque extérieure (11).

19. Cupule d'articulation modulaire selon l'une des revendications 15 ou 17 ou 18,
**caractérisée**
**en ce que** l'insert (12) est réalisé en polyéthylène UHMW élastique.
